# EUROPEAN PATENT APPLICATION

(11) **EP 0 919 194 A1**
(43) Date of publication of application: **02.06.1999**
(21) Application number: 98203991.9
(22) Date of filing: 23.11.1998
(51) Int. Cl.: A61B 17/04, F16B 13/06

(54) **Device for anchoring a suture to bone**

(30) Priority: 25.11.1997 IT PN970037
(71) Applicant: Cerruti-Quara, Piero, 33090 Arzene (PN) (IT); Muollo, Ferdinando, 84018 Scafati (SA) (IT)
(72) Inventor: Cerruti-Quara, Piero, 33090 Arzene (PN) (IT); Muollo, Ferdinando, 84018 Scafati (SA) (IT)
(74) Representative: Da Riva, Ermanno

(57) **Abstract**

Anchorage device particularly on bony parts, of suture means, composed by two fondamental elements (1-2), both made up by respective cylindrical tubular elements (10-20) provided of longitudinal cavities (12-22) generating respective fingers (13-23) which complement themselves so as to be in opposition. The fingers (13-23) penetrate into opposite corresponding longitudinal cavities (12-22). The two fondamental elements (1-2) are applied on an operating shaft (3) between an enlarged part (32) and a pushing element (4). In operation, the said fondamental elements (1-2) are urged against on another by an application tool so that the fingers deflect outwardly and fasten the device securely in the hole. A suture threads (S) can then be inserted in a slotted hole (39) provided into the extremity of the leg (312) of the operating shaft.

## Description

The object of this discovery is a dispositive to be used essentially in the medical-surgical field, to link together tissues and bones, and even more to fix the various prosthesis to the relevant bony parts. The dispositive in object is particularly used to form an element that can be fitted into the relevant hole made in a bone to which it can be easly fixed, permitting the sicure anchorage of the suitable elements of suture used to fix to the same bone those tissues that for surgical reasons or due to various accidental causes have been loosed.

Many different dispositives are known which can be fitted in and fixed in various ways into a blind hole made in the relevant bone part to allow the linking and the fixing of other elements, as tendoneus tissues or tissues of other nature, or also of further bony parts and even more various prosthesis. It is particularly known a dispositive of the kind of the one in object that results to be described in the U.S. Patent N. 5.645.589 and even more the patents to which this one refers and exactly the U.S. Patent N. 2.143.086-U.S. Patent N. 2.100.570-U.S. Patent N. 5.161.916-U.S. Patent 1.247.621-U.S. Patent N. 4.293.259-U.S. Patent N. 5.094.563-U.S. Patent N. 34.293-U.S. Patent N. 5.037.422, their limitations and inconveniences are explaned and commented in this patent and therefore they will be not repeated.

Anyway also this U.S. Patent N. 5.645.589 presents various inconveniences, moreover in different, also essential parts, the described and illustrated solution does not correspond to the practical realization that has been commercialized by the firm, holder of this patent. The unique parts of the product that can be aproximately considered correspondent to part of the elements that have been described and illustrated in this patent are only the two elements provided of fingers, which are so disposed that those of one element penetrate the interspaces resulting between the fingers of the other and so conformed, that a generical strength that pushes these elements one against the other, provoques a deformation and the relevant radial deplacement of every finger to the external in order to penetrate into the hole surface, that has been made in the body in which we want obtain the passage of the anchorage dispositive and to which this dispositive of anchorage was previously fitted in. It is to notice that similar solutions, based on the same principle are further known.

Besides in a unique patent, different variations are described, so different one from the other to generate a big confusion.

Among other things a solution is known, contemplating that the fingers of one of the opposite elements, rather than to be opened to the external, are folded to the inner side, this results incomprehensible both considering the fact that it is not clear which effect of anchorage should result and because it is inexplicable the system that can be adopted, to get the type of deformation. On the whole in this patent are included so many variations, among them incongruous and incomprehensible that do not permit to understand exactly both their function and their working and even not the driving system and/or systems. The unique evident thing is the fact that they have subdolously and very confusedly reunited pratically in one patent, if not all possible variations of realization and application, at least a big quantity of solutions among them totally different and even confusedly or too scantily and insufficently explaned, with the unique scope to prevent the other from any possible solution. Apart from the content of this patent, we consider the product that derives and that is commercialized through it, to be that further, substantially different from what is in it in generic terms and confusedly described, claimed and illustrated, presenting in its known commercial version, both functional and applicable inconveniences and limitations. First of all the radial opening of the fingers is limited, beside the conformation of their inner surface forms a sharp corner and their extremity results sharp therefore when these fingers are radially open to the external, although they penetrate into the material of application, normally a bone, they permit a limited anchorage that sametimes does not result reliable. Another inconvenience derives from the fact that in the first form really resulting from the practical realization, that as told is different from the description explaned in the patent, the stem that is inserted between the two elements presenting the opposite fingers in order to provoque, through the use of an idoneous application utensil, the opening of the relevant fingers to the external is cylindrical and penetrates in the inner cylindrical holes of these elements without any conformation able to avoid the free rotation into the same.

It is helpful and opportune to prevent this rotation because at the end of the operative phase that has provocated the expansion of the fingers of both elements, the operator in order to take away from the compression stem the extremity fixed to the application utensil, practises beside the traction produced with this instrument, also a rotation that helps the separation.

Further, the extremity of this compression stem that by the anchorage is located at the bottom of the hole made in the bone is formed of a conical point that obviously can freely rotate on the walls of this bottom, seen that it is also smoothy, when it is opportune that it seizes itself on it without rotating.

It is also to notice that according to the patent to which we relates, the compression strength that should produce the expansion of the fingers of anchorage is obtained through the traction practised from the suture that should pass into the hole considered on the inner side of the stem, inserted in the two elements that present these fingers.

This solution is obviously at least disputable seen that, as already mentioned, it has been changed in the practical realization. To obviate the inconveniences and limitations of the various kind of the known dispositives of anchorage and especially of the effective dispositive that should correspond to the above mentioned U.S. patent N. 5.645.589, it has been studied and realized the dispositive of anchorage that is object of the following discovery, to improve the understanding of the characteristics and the advantages that can be obtained through it, only for an explicative and not limitative reason, it is here below described with reference to the enclosed drawings in which :
- fig.s 1 and 2 show with front views the two fundamental elements of the dispositive, object of the present discovery, in the position preceding the application,
- fig.s 3 and 4 are transversally sectioned views of the correspondent elements, rapresented on fig.s 1 and 2,
- fig. 5 is a sectioned view according to the line I-I of fig. 1 or according to the line II-II of fig. 2
- fig. 6 is a view taken from the inferior part of the element of fig. 4
- fig.s 7 and 8 are two front views, among them orthogonal, showing the stem utilized for the driving of the elements shown on figures 1 and 2 for the anchorage of the suture
- fig.s 9 and 10 are views of the stem shown on fig.s 7 and 8
- fig. 11 shows with a front view the whole dispositive inclusive of the driving element, in the position preceding the application.
- fig. 12 is a front view orthogonal to the previous fig. 11, that for clarity includes a brocken part
- fig. 13 shows through a sectioned view, the completed dispositive and the relevant final positioning of anchorage
- fig. 14 shows as by fig. 13, the only dispositive fixed on the material of application and ready to be used.

In these figures the common parts are called with the same references. With reference to the fig.s from 1 to 6 the basic elements 1 and 2 used to obtain the desired anchorage are described. As from fig.s 1, 2, 3 and 4 the two basic elements 1 and 2 are especially similar and result to be formed from the relevant tubular cylindric elements, 10 and 20 that have a length ca. equal to their external diameter, in each of them are made the longitudinal cavities 12 and 22 that radially penetrates into the correspondent inner holes 11 and 21, these cavities are equidistanted one from the other and result to extend themself for a tract equal to 2\3 of the length of the correspondent basic elements 1 and 2.

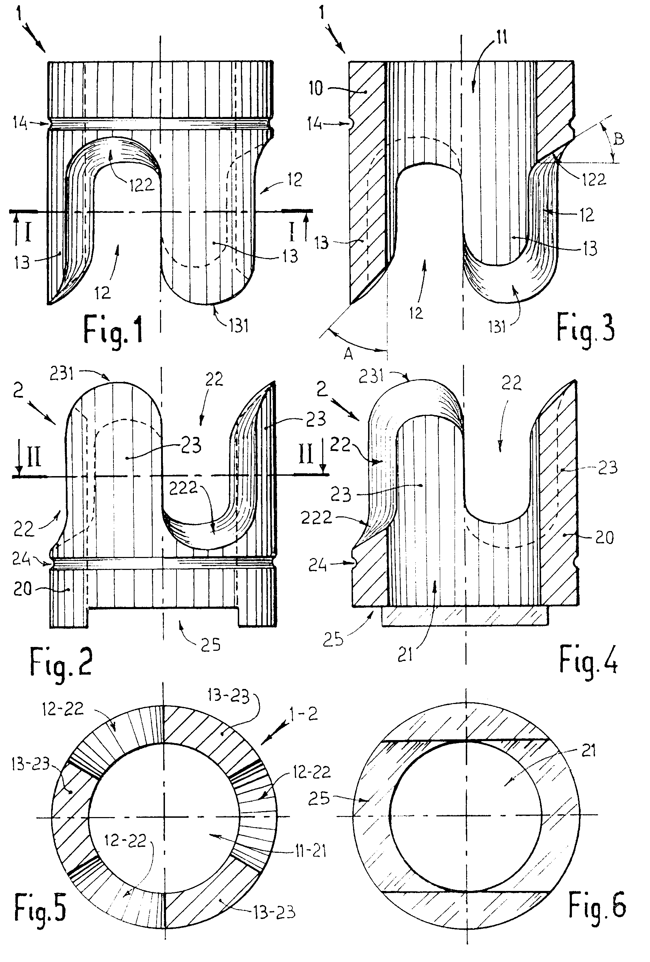

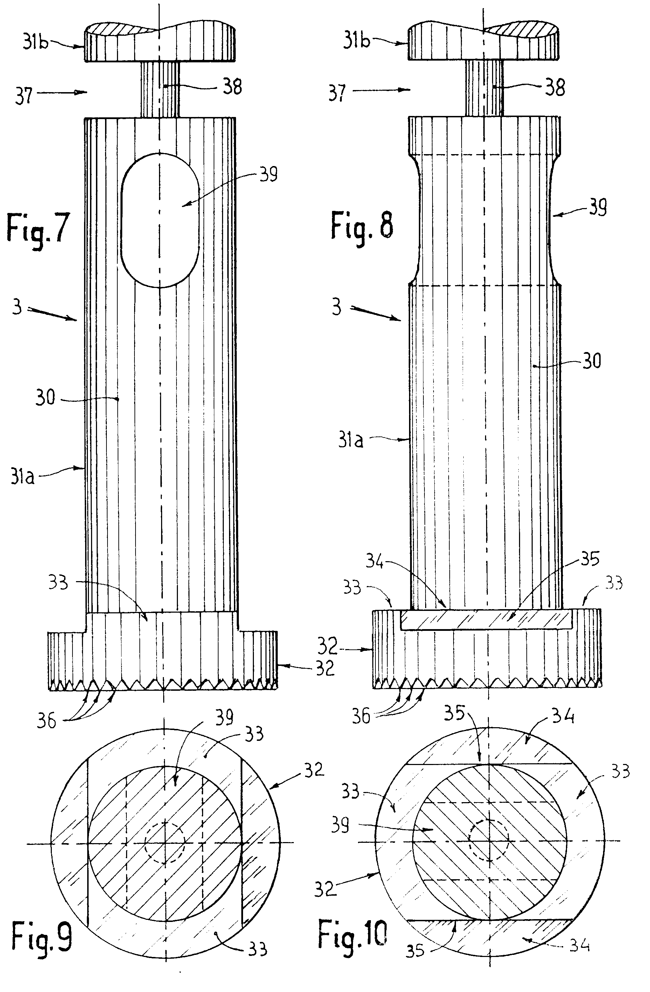

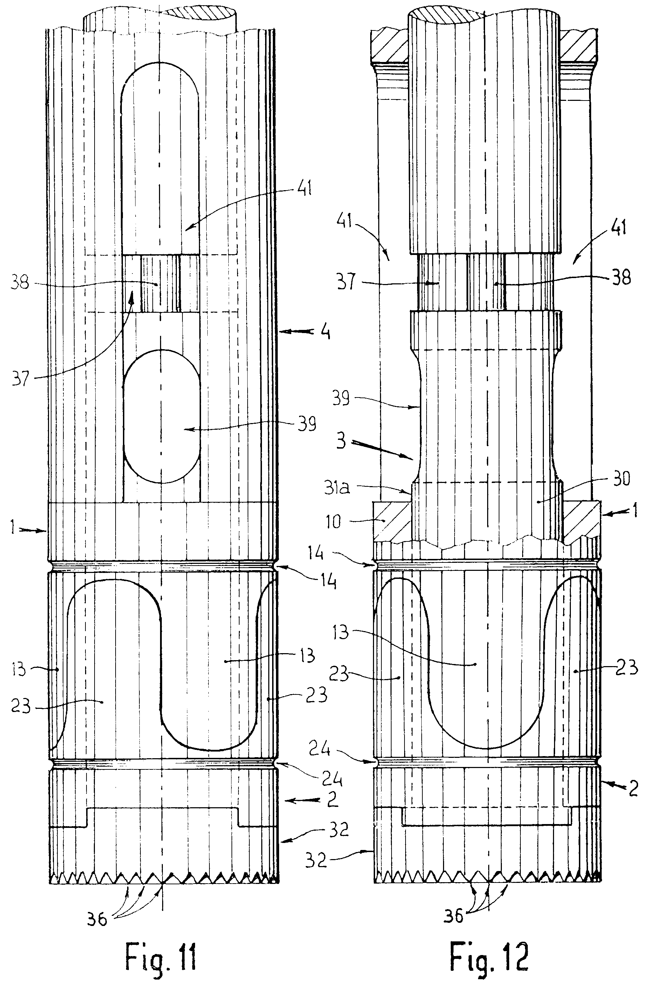

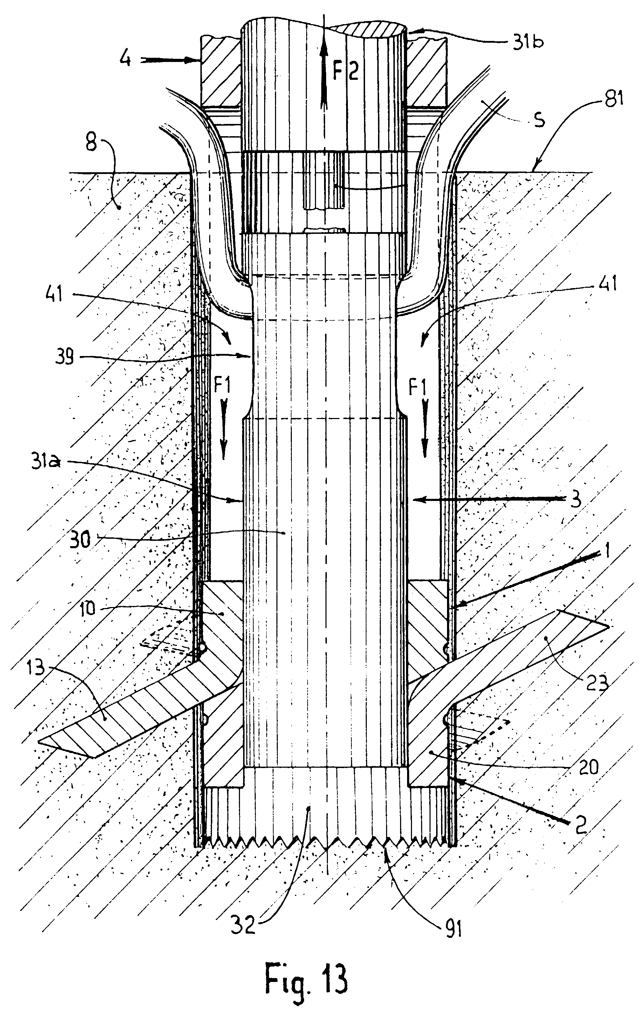

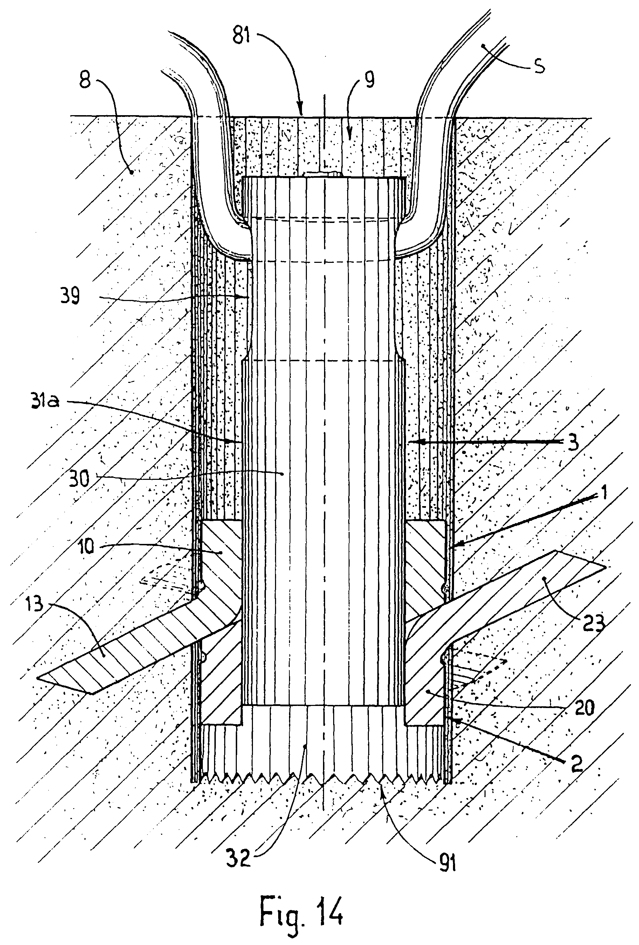

Beside these longitudinal cavities present equal widths to the filled parts resulting between them, generating in this way the relevant longitudinal elements that form the relevant fingers 13 and 23. This fingers 13 and 23 terminate on their free ends with the correspondent tracts 131 and 231, substantially with a semicircular form and as especially evident on fig. 3, they penetrate in an inclined way converting to the inner side of the relevant basic element 1 and 2 with an angle A of 45 degrees ca. Also the inner extremities 122 and 222 of this longitudinal cavities 12 and 22 have a semicircular form and the correspondent surfaces are also inclined converting to the inner side but to form, as also especially evident on fig. 3 an angle B, that for the reasons below reported, results preferably discreet inferior to the angle A (30 degrees ca.).

The two element 1 and 2 result to be complementary one to the other, therefore as shown on fig.s 11 and 12 the respective fingers 13 and 23 can penetrate into the correspondent longitudinal cavities 12 and 22 of the opposite element. Finally on the external surface of each of this tubular cylindric elements 10 and 11 are obtained in the proximity of the inner extremities 122 and 222 the respective longitudinal cavities 12 and 22 and then anchorage areas from which the correspondent fingers 13 and 23, the relevant annular flutings 14 and 24 with a form preferably semicircular, further described, depart.

The basic element 2 deters from the basic element 1 for the fact that on its opposite extremity to that from which the respective fingers 23 depart, a transversal cavity 25 is realized, with a form essentially rectangular that penetrates discreetly into it and that presents a width equal to the diameter of its inner hole 21, this transversal cavity 25 results to form the female part in which the correspondent male parts 33 obtained in the driving stem 3 can get engaged, as below explaned. As from the following fig.s 7, 8, 9 and 10 this driving stem 3 is formed of a cylindric element 30 that develops itself for a discreet part opportunately bigger respect to the whole length, that results from the combination of the mentioned basic elements 1 and 2. (See fig.s 11 and 12) This cylindric element 30 ends in the extremity that, as from fig.s 13 and 14, by the application results to be disposed on the bottom 91 of the hole 9, made in the application material 8 (normally a bony element), with the head 32 formed of a cylindric enlarged part, presenting a diameter equal to the external diameter of these basic elements 1 and 2. In the part where the head 32 joints itself to the cylindric element 30, two lateral facings 34 that generate two flat inner sides 35 tangent to the external surface of the cylindric element 31 have been made on the sides of this (32), parallelly opposite one to the other. These lateral facings 34 form among them two protruding parts forming respectively the male parts 33, complementary to the generated female part, as previously explaned, from the transversal scoop 25 that is obtained in the basic element 2 in which they can penetrate.

The extremity of this head 32 that during the application will draw against the bottom 91 of the hole 9, obtained from the application material 8, (see fig.s 13 and 14), will be provided, in the possible realization form to which we refer, with a plurality of radial facings, generating the correspondent protruding radial elements 36 with a triangular form, capable to get engaged with this application material 8 in order to avoid the rotation of this driving stem 3. Obviously this conformation can vary, for ex. instead of the protruding radial elements 36 a facetted point can be made, for example a pyramidal point with a polygonal bottom or a lanceolate point or other possible conformations capable to obtain the same scope. On the cylindrical element 30 forming this driving stem 3, at a distance from the head 32 opportunely bigger respect the defined length of the whole of the basic elements 1 and 2, is realized a deep annular fluting 7 that separates the part 31a, forming the part that with the basic elements 1 and 2 applied on the same, at the end of the application remains anchored to the application material 8, from the part 31b that forms an adequated part able to be fixed into an idoneous application utensil of the known kind. In this way the two parts 31a and 31b result connected only from a thin cylindric element 38, beside on the part 31a, closed to this annular 10 fluting 37 a hole 39 is made to allow the penetration in it of at least a suture thread S.

When we have finished with the description of the anchorage dispositive in object, we proceed with the explanation of its application. On the driving stem 3 are inserted at first the basic element 2, disposed in such a way that its female part 25 and its male parts 33, obtained on the same driving stem 3 can respectively penetrate into, then the element 1, in such a way that its fingers 13 penetrate into the longitudinal cavities 22 of the previous basic element 2 and at the same time the fingers of the same penetrate into its longitudinal cavities 12.

The part of the driving stem 3 is fitted in, this stem is protruding from the group of the basic elements 1 and 2 so assembled, into the inner hole of the extremity of a pushing element 4 that results interplaced between the application utensil and the same basic element 1, allowing also the contemporaneous penetration of an adequated part of its free extremity into the traction element of this application utensil to which it will be fixed.

The pushing element 4 or the part of the driving stem 3 that is inserted in it will be provided, as known, with an opportune driving system, (not shown here), able to permeet only the longitudinal sliding of this driving stem 3 avoiding any possible rotation.

The extremity of the pushing element 4, as by fig.s 11 and 12 is formed of a tubolar cylindric part 41 that presents an external diameter equal to that of the basic elements 1 and 2 and it is provided with two longitudinal cavities 41, diametrically opposite one to the other that have a width at least equal to that of the hole 39 and that develop itself for a part that is superior respect the area of the annular fluting 37 of a length discreetly superior to the pressing run of the basic elements 1 and 2. These longitudinal cavities result obviously placed along the opposite openings of the hole 39.

In the desired area of the application material 8 that has mentioned is normally formed from a bony part, with an adequated utensil the is made the hole 91 that presents a diameter at least equal or preferably lightly superior respect the external diameter of the basic elements 1 and 2 and a depth capable to contain at least the whole group of anchorage, (basic element 1 and 2 with the relevant head 32 of the driving stem 3 in such a way that on the external side of the surface of the application material protrudes only the area of the part 31a of the driving stem 3 that ends into the annular fluting 37.

Before the insertion into the hole 91, the suture thread (or threads) S will be applied to the anchorage dispositive, fitting it into the hole 39, preferably with the help of an idoneous utensil of the type of the one known used to thread the needle. To this point the completed dispositive of anchorage connected to the application utensil, is fitted into this hole 91 that is made as previously described, in the application material 8, then opportunatly driving the application utensil this provocates, in the traditional and known way, on the pushing element 4, a direct push as shown in the fig. 13 from the farrows F1, to the group of the basic elements 1 and 2 and to the driving stem 3, as shown in fig. 13 from the farrow F2 in the opposite direction. Subsequently the mentioned basic elements 1 and 2 result reciprocally pushed one against the other so that the parts 131 and 231, resulting at the extremities of the relevant fingers, 13 and 23 slide with the respective inclined inner parts 122 and 222 of the longitudinal cavities 12 and 22, diverting in this way to the external the fingers 13 and 23. Pursuing the movement of mutual compression of the same basic elements 1 and 2, also the inner parts of the fingers 13 and 23 will slide on the inclined parts of the inner extremities 122 and 222, so that they will progressively expand penetrating contemporaneously into the application material 8. The movement will carry on till the inner parts resulting on the bottoms of the fingers 13 and 23 will pratically drawn to the inclined surfaces of the opposite inner extremities 122 and 222 of the correspondent longitudinal cavities 12 and 22. It is to notice that the sliding of the fingers 13 and 23 pratically takes place through the contact to the external angle of the inclinated surfaces that result on the relevant opposite inner extremities 122 and 222, therefore at the end of the scarf of these basic elements 1 and 2, seen that these surfaces are restrictly inclinated, (about 30 degrees) respect to the inclination that is normally used, (about 45 degrees), the fingers 13 and 23 will result disposed in penetration into the application material 8 in a discreetly closed way one the other, that permits a better anchorage and a better resistance to the tear. Beside the realization at the bottom of the fingers 13 and 23 of the annular flutings 14 and 24, reducing the thickness of the resistent material on the correspondent areas in which the inflection of the same fingers 13 and 23 is produced, the folding of these results agevolated therefore an inferior effort of compression is required and subsequently the resisting section of the cylindric element 38 can be reduced, therefore for its tear is required minor strength and then a minor driving effort of the application utensil, that results very helpful and profitable because beside agevolating the operation it reduces, even practically eliminates the causes of rupture that offen occures to the utensils of this kind due to the remarkable exertions to which they are subjected.

Beside, seen that the basic elements 1 and 2 and the driving stem 3 are interconnected in the way explaned that prevents to the same any possible rotation, the eventual movement of rotation, that the operator generally impresses to obtain or to better facilitate the separation of the part 31b of this driving stem 3 from the other part 31a of the same that results to be fixed with the basic elements 1 and 2 on the application material 8, results especially agevolated and anyway it requires, if necessary, only a light and very reduced effort.

When the fixing operation of the dispositive in object has taken place in the body of the application material 8 and when the separation from the same of the rimanent external part and exactly of the tract 31b of the driving stem 3 that remains connected to the application utensil and to the pushing element 4 is obtained, the group of anchorage formed by these basic elements 1 and 2 and the relevant part 31a of the driving stem 3, remains safely anchored to the application material 8.

By the end of the operation we will have the disposition shown in the fig. 13. To this point it is possible to remove the application utensil removing the part 31b of the driving stem 3 and the relevant pushing element 4, so that, as shown in the fig. 14, remains inserted only group of the basic elements 1 and 2, the fingers 13 and 23 of the same, radial expanding themself as described, penetrating into the correspondent circunferential wall of the hole 9 inserting itself in the body of the circumstantial application material 8 and obviously in the part of the application stem 3 conprehensive of the relevant part 31a and the relevant head 32.

The part of the extremity of this part 31a that includes the hole 39 in which was inserted the suture thread (or threads) S remains also discreetly inserted in the inner side of the hole 9 resulting positioned with its external extremities, opportunely placed under the external surface 81 of the application material 8. It is then possible to make the suture of the tissues or of other organical materials or of other kinds as for ex. materials of prosthesis structure that result fixed to the application material 8. It is to notice that the material used to make the various elements of the dispositive in object is obviously of the biocompatible type as for ex. a suitable inox steel or other suitable metals and for particular applications also suitable plastic materials. Preferably, as texted during practical experimental applications, the material that is resulted more suitable is a particular alloy of titanium (ELI).

Beside is very important the treatment of the surfaces of the parts that get in contact with the application material 8, as bones and/or various organical tissues, in fact for obtaining the best holding of the surfaces of the parts and the organic material to which they get in touch it is opportune that these are made adequately rugose. The required rugosity can be obtained with various procedures and elements, as the well known realization of covers obtained through the system of "plasma spray" that anyway is of difficult execution, it requires special instruments and subsequently it results to be very expensive, or through electroerosion procedures that far to bring unsatisfied results it requires always special instruments and notable times of realization.

The system particularly applied during the sperimental executions that have been tested and that has given the best results, is the well known procedure of sand-bathing performed with particular kinds of idoneus abrasive powders of adequated granulometry.

From what has been explaned are evident the remarkable applicative and functional advantages that the dispositive in object apports respect to the similar and well known dispositives, the principal of them are here below listed.

They are :
a) better anchorage and holding to the application material (bones or other organical materials)
b) remarkable reduction of the application efforts
c) impediment of every possibilities of rotation of the application stem with subsequent elimination of the drawbacks that can derive from it
d) executive semplicity of the various parts of the relevant assembling beside their application
e) excellent cohesion between the anchorage parts and the material into which they are fitted.

The drawbacks and limitations of the similar and well known dispositives are avoided.

Obviously the applications of the described dispositive can widthly variate, further it is easly understandable that many variations beside the one already explaned can be apported to the parts of these dispositives without deserting from the ambit of what has been described and here below claimed with reference to the enclosed drawings and therefore to the dominion of protection of the present industrial patent.

## Claims

1. Anchorage dispositive of suture elements especially for bony parts, this dispositive results essencially applicable to the surgical-medical field to fix into a hole, obtained from a part of an application material as normally a bone or other element of the body of an alive, one of its part to which are connected suitable elements of suture that in this way result to be fixed, allowing the relevant fixing of other various materials as organic tessius or prosthesis or others, this dispositive is formed of two basic elements (1-2) formed from respective tubolar cylindric elements (10-20) in which are obtained longitudinal cavities (11-12) that are equidistant one to the other, that radially penetrate from one of their extremity, extending themself for an adequated part, forming in the full parts that result between them, the respective fingers (13-23) that are complementary conformed to the same (11-12), the inner extremities (122-222) of these longitudinal cavities (12-22) and the free extremities (131-231) of these fingers (13-23) result totally bent converging to the inner side of the respective basic elements (1-2), these basic elements (1-2) are placed in such a way that the respective fingers (13-23) and correspondent longitudinal cavities (12-22) compenetrate each other, these basic elements (1-2) that are fitted in a driving stem (3) in such a way that one of their extremity draws against an enlarged extremity of the same forming the relevant head (32), result so included between this (32) and a pushing element (4) that is interposed between the other extremity and an idoneus application utensil to which the free extremity of this application stem (3) is fixed, the functioning of this application utensil results contemporaneously provoking a traction to the driving stem (3) and a push in the opposite way on the push element (4) so that the resulting compression, that devolops on these basic elements (1-2) provoques the mutual approach that causes a progressive sliding of the fingers (13-23) to the inner extremities (122-222) of the relevant longitudinal cavities (12-22) that result to be bent to the inner side provoking a correspondent opening of these fingers (13-23) and subsequently their progressive penetration into the lateral wall of the hole (9), previously made in the correspondent part of the application material (8) in which the dispositive in object has been fitted and that so results permanently fixed, the part (31a) of this driving stem (3) to which the basic elements (1-2) have been applied, results discreetly protruding from them with a part on which is made a hole (39) able to contain the idoneus suture threads S and results to be separated from the successive part (31b) that gets engaged into the application utensil, from a deep annular fluting (37) generally central respect to a thin cylindric element (38) so that when the operation of expansion and the fixing of the basic elements (1-2) are finished, an ulterior traction applied from this application utensil provoques the breakage of this thin cylindric element (38) with the subsequent separation of this part (31b) that in this way can be removed through the application utensil to which it remains applied and the relevant pushing element (4), this all resulting substantially known; the anchorage dispositive, that is the object of the present discovery characterized by the fact that at least one of the two basic elements (1-2) are conformed substantially in an equal way, results provided of a part (25) able to get into the correspondent parts (33), obtained on the enlarged part forming this head (32) of the driving element (3) on which these basic elements (1-2) have been fitted, so to avoid any possibility of mutual rotation; the free extremity of the fingers (13-23) and the inner extremities (122-222) of the longitudinal cavities (11-12) in which penetrate the fingers (13-23), develope into respective arched parts (131-231) convergently bent one to the other to the inner sides of the correspondent basic elements (1-2); finally close to the inner extremities of these longitudinal cavities (12-22) and then of the areas from which the relevant fingers (13-23) depart, are obtained the relevant annular flutings (14-24) able to adequately reduce the resistant section of the relevant anchorage sections of these fingers (13-23).

2. Dispositive as by claiming 1, characterized by the fact that the external extremity of the head (32) of this driving stem (3) is provided of protruding elements that are adequately conformed, able to get engaged to the bottom (91) of the hole (9) obtained in the application material (8) so to avoid any possibility of rotation of this driving stem (3) and subsequently of the group of the two basic elements (1-2) applied on it, into the hole (9) in which they have been inserted;

3. Dispositive as by claiming 2 characterized by the fact that these protruding elements, obtained on the external extremity of the head (32) of the driving stem (3) can be realized through a plurality of radial flutings (36) with a triangular or similar section able to generate the correspondent protuding sharp-parts.

4. Dispositive as by claiming 2 and 3 characterized in the fact that these protruding elements can present a pyramidal point with a polygonal base or a lanceolate point or equivalent.

5. Dispositive as by claiming 1 characterized by the fact that the inclinations of the free extremities of the fingers (13-23) unroll themself departing from the external surface of the relevant basic element (1-2) directed to the inner side of the same, forming an angle (A) of 45 degrees ca., while the inclinations of the inner extremities (122-222) of the relevant longitudinal cavities (12-22) totally penetrate into the previous parts with an angle (B) discreetly minor, equal to 30 degrees ca.; this difference permits, during the execution of the compression operation of these basic elements (1-2), that their fingers (13-23) slide with the rispective inner parts on the correspondent external margins of the opposite inner extremities (122-222) of the correspondent longitudinal cavities (12-22), obtaining at the end of the operation a bigger opening of these fingers (13-23) and contemporaneously a better anchorage into the application material (8).

6. Dispositive as by claiming 1 characterized by the fact that these annular flutings (14-24), obtained on the basic elements (1-2) in correspondence of the anchorage areas of the respective fingers (13-23), present preferably a semicircular form able to connect the relevant parts that during the inflexion to the external side of these fingers (13-23), bent to shorten in such a way that they come to a more razional distribution of the solicitations, that together with the reduction of the correspondent resisten sections, allows the notable reduction of the compression efforts that must be imprexed to these basic elements (1-2) to provoque the opening of the relevant fingers (13-23). All this as substantially explaned and claimed with reference to the enclosed drawings and for the desired scopes.
